# EUROPÄISCHE PATENTANMELDUNG

(11) **EP 0 919 227 A1**
(43) Veröffentlichungstag der Anmeldung: **02.06.1999**
(21) Anmeldenummer: 97121045.5
(22) Anmeldetag: 01.12.1997
(51) Int. Cl.: A61K 9/20, A61K 35/78

(54) **Brausezubereitung enthaltend ein Pflanzenextrakt**

(71) Anmelder: Gergely, Gerhard, Dr., A-1050 Wien (AT)
(72) Erfinder: Gergely, Gerhard, Dr., 1053 Wien (AT); Gergely, Irmgard, 1053 Wien (AT); Gergely, Thomas, Dr., 1053 Wien (AT); Gergely, Stefan, Dr., 1053 Wien (AT)
(74) Vertreter: Büchel, Kurt F., Dr.

(57) **Zusammenfassung**

Eine Brausetablette enthält wenigstens einen wasserlöslichen oder zumindest suspendierbaren Pflanzenextrakt und eine Brausebasis. Die Pflanzenextraktteilchen liegen in inniger Mischung mit einem wasserlöslichen, pharmazeutisch zulässigen Füllstoff vor, der weniger schnell bzw. weniger leicht löslich ist als Zucker, insbesondere Maltodextrin, Mannit und/oder Lactose. Die Brausebasis ist in ihrer Zusammensetzung und Menge so eingestellt, dass sich mit dem Pflanzenextrakt in wässriger Lösung ein pH-Wert von 3.8 bis 5.6, insbesondere von 4.1 bis 5.1, ergibt, am besten durch ein Gewichtsverhältnis der sauren zu den CO2-abspaltenden Komponenten der Brausebasis zwischen 1:0.3 und 1:0.9, bzw. ein Gewichtsverhältnis des Pflanzen-Trockenextraktes zur Brausebasis zwischen 1:3 und 1:25, bevorzugt zwischen 1:5 und 1:20. Die Brausezubereitung ist vorteilhaft für Pflanzenextrakte wie Silymarin, Cimicifuga, Echinacea, Efeu oder Agnus Casti und enthält, insbesondere im Fall von Echinacea, Spurenelemente, z.B. in Form einer wasserlöslichen Zinkverbindung, bevorzugt etwa 5 bis etwa 50 mg Zinkion pro Tablette.

## Beschreibung

Die Erfindung betrifft eine Brausezubereitung nach dem Oberbegriff des Anspruches 1. Fast alle Pflanzen-Trockenextrakte beeinflussen die Auflösung, allerdings - in Abhängigkeit von den charakteristischen Inhaltsstoffen - mehr oder weniger stark. Einige Pflanzenextrakte erfordern aufgrund ihrer stark verzögernden Wirkung der Auflösung in Löstabletten oder Brausetabletten ganz besonderer Maßnahmen, um diese Eigenschaften zu kompensieren. Das Problem der Verschleimung von Pflanzenextrakten, die deshalb schlechte Auflösungseigenschaften in wässriger Lösung zeigen, wird z.B. für eine solche Brausezubereitung in der EP-A1 97118648.1 dadurch umgangen, dass die Pflanzenextrakt-Teilchen mit einer dünnen Schicht einer öligen, fettigen oder wachsartigen Substanz beschichtet werden. Dies ist aber ein zusätzlicher Arbeitsschritt, und ist auch wegen des Einbringens solcher Substanzen in die zum Trinken bestimmte Brauselösung nicht in allen Fällen erwünscht.

Es gibt jedoch auch andere Trocken-Extrakte, wie Silymarin, Cimicifuga, gering dosierte Efeuextrakte und Echinacea-Extrakte, die zwar die Auflösung einer löslichen Darreichungsform, wie einer Lös- oder Brausetablette, geringfügig verlangsamen, jedoch nicht in einem solchen Ausmaß, daß die erwähnten, extremen Maßnahmen erforderlich sind.

Nun hat man auch schon vorgeschlagen, dem flüssigen Pflanzenextrakt vor seiner Trocknung Zucker zuzusetzen (EP-A1 743 062), oder man hat sich mit der Mischung von Pflanzenextraktteilchen mit den Komponenten der Brausebasis (WO 97/2064) zufrieden gegeben. Beides ergibt keine oder nur eine beschränkte Lösung des eingangs geschilderten Problems: Zucker ist einerseits aus Kaloriengründen in pharmazeutischen Zubereitungen eher unerwünscht; andererseits ist er leicht und schnell löslich, wodurch das Verschleimen eher noch begünstigt wird. Ebenso leicht und schnell sind die Komponenten der Brausebasis löslich. Darüberhinaus haben beide genannten Brausezubereitungen der Zusammensetzung und Menge der Brausebasis kaum Bedeutung beigemessen. Diese ist aber für eine gut lösliche, Pflanzenextrakt enthaltende Brausetablette von grösster Bedeutung.

Die wesentlichsten Darreichungsformen für Phytopharmaka sind derzeit Tabletten, Dragees oder Tropfen, wie z. B. für Echinacea, Silymarin, Efeu u.a.

Die Erfindung hat sich nun die Aufgabe gestellt, eine lösliche Darreichungsform für Pflanzen-Trockenextrakte zu entwickeln, die alle erwähnten Nachteile vermeiden und sich in Wasser von 15 bis 18°C innerhalb von höchstens 3 min auflösen. Diese Aufgabe wird erstmals in überraschender Weise durch die Kombination der im Kennzeichen des Anspruches 1 genannten Merkmale gelöst. Details und Weiterbildungen des Erfindungsgedankens sind in den abhängigen Ansprüchen beschrieben.

Einerseits sollen die Pflanzenextrakt-Teilchen mit solchen pharmazeutisch zulässigen Hilfs- bzw. Füllstoffen versetzt werden, die zwar löslich sind, sich aber nicht so schnell und leicht wie Zucker lösen dürfen, da sie dann das Verschleimen der Pflanzenextrakt-Teilchen und damit eine Verlangsamung der Auflösung nicht oder zu wenig verhindern. Andererseits sollen sie auch nicht so schwer löslich wie Dextrin sein, weil im Falle der entsprechend erforderlichen Menge von Dextrin keine klare Lösung erzielt werden kann und andererseits das Aufziehen von fettig, ölig oder wachsartigen Substanzen einen zusätzlichen Arbeitsschritt bedeutet und technologisch insofern aufwendig ist, als man mit organischen Lösungsmitteln arbeiten muss und dieser Schritt bei einer Reihe von Pflanzenextrakten nicht unbedingt erforderlich ist, wenn man eine Zusammensetzung entsprechend dem Erfindungsgedanken wählt.

Als besonders zweckmässig im Sinne der Erfindung haben sich in diesem Zusammenhang Mannit und/oder Lactose erwiesen.

Andererseits hat sich gezeigt, daß man durchaus zufriedenstellende Auflösungseigenschaften und Auflösungszeiten dann erhält, wenn man die Extrakte mit einer Brausebasis aus CO2-abspaltenden Substanzen und eßbaren, organischen Säuren, bzw. deren Salzen vermischt, wobei das Verhältnis von Säuren bzw. deren Salzen zu den CO2-abspaltenden Substanzen 1:0,3 bzw. 1:0,9 beträgt, sodass ein pH-Wert von 3,8 bis 5,6 resultiert. Als alkalische, CO2-abspaltende Bestandteile können sämtliche Alkalihydrogencarbonate und/oder - carbonate, wie Kaliumhydrogencarbonat, Kaliumcarbonat, Natriumhydrogencarbonat, Natriumcarbonat, Natriumglycincarbonat u.ä. eingesetzt werden, als Säuren insbesondere Zitronensäure, Weinsäure, Äpfelsäure und/oder deren sauren Salze. Sowohl die Alkali- als auch die sauren Komponenten können entweder einzeln oder auch in Mischung vorliegen.

Eine zweite wesentliche Komponente der Erfindung ist das Verhältnis des Extraktes zu der Gesamtmenge der Brausebasis, das - um eine Auflösungszeit von höchstens 3 min zu erreichen - ein Verhältnis von 1 Gewichtsteil Pflanzen-Trockenextrakt zu 3 bzw. 25 Gewichtsteilen der Brausekomponenten aufweist. Verhältnisse, bei denen der Anteil der Brausekomponenten in der fertigen Darreichungsform wesentlich über 25 Gewichtsteilen liegt, bringen interessanterweise keine signifikante Beschleunigung der Auflösung, sondern es resultiert nur ein - in der Regel unerwünschtes - größeres Tablettengewicht und damit auch eine weniger rationelle Herstellung, und zwar insbesondere aufgrund des höheren Rohstoffeinsatzes und der damit unwirtschaftlicheren und teureren Formulierung. Werden andererseits in der Rezeptur weniger als 3 Gewichtsteile Brausebasis auf 1 Gewichtsteil Extrakt eingesetzt, so macht sich dies bereits in einer signifikanten Verlangsamung der Auflösungszeit durch den Einfluß der Pflanzen-Trockenextrakte bemerkbar.

Z.B. zeigt eine Echinacea-Brause- bzw. Löstablette, bei der weniger als 3 Gewichtsteile Brausebasis auf 1 Gewichtsteil Extrakt vorliegen, bei einer Härte von 7,5 bereits eine Auflösungszeit von über 3 min, während eine erfindungsgemäß hergestellte Brausetablette (mit einem Verhältnis von Echinacea-Trockenextrakt zu Brausebasis 1:10) eine Auflösungszeit von nur 80 sec bis 1 ½ min zeigt. Beträgt das Verhältnis der Brausebasis zum Echinacea-Trockenextrakt jedoch mehr als 25 (bei gleichem Tablettengewicht und gleicher Härte), so ergibt sich dabei immer noch dieselbe Auflösungszeit von ca. 80 sec. Man sieht somit, daß selbst bei einer auf etwas mehr als das 2 1/2-fache vergrößerten Brausebasismenge keine wesentlich schnellere Auflösungszeit resultiert. In diesem Fall befindet sich aber bei gleichem Tablettengewicht nur mehr ein kleiner Teil der Extraktmenge in der Tablette, z.B. statt 250 mg nur mehr 105 mg. Somit müssten 2 ½ Tabletten verabreicht werden, um die entsprechende Dosis zu erzielen. Bei gleichbleibender Dosis und bei einem Verhältnis von 1 Teil Trockenextrakt zu über 25 Teilen Brausebasis würde bei einer Dosierung von 250 mg Trockenextrakt pro Dosis der Brausebasisanteil bereits über 6 g betragen und wäre somit nicht mehr wirtschaftlich und teuer.

In einem pH-Bereich über 5,6 erfolgt die Reaktion zwischen den Säurebestandteilen und den Alkalibestandteilen des Brausesystems bereits langsamer, sodaß durch den zusätzlichen Einfluß der Pflanzenextrakte die Auflösungszeit weiter verlängert wird. Bei einem saureren pH-Wertals 3,8 ist wieder nicht genügend CO2-abspaltender Alkalibestandteil vorhanden, um den verzögernden Einfluß der Trockenextrakte auf die Auflösungszeit durch eine vehemente Reaktion der beiden Brause-Bestandteile zu kompensieren.

Bei einer Echinacea-Brausetablette, mit einem Verhältnis von Echinacea-Trockenextrakt zu Brausebasis von 1:6,5, die ein pH-Wert von 3,6 zeigt, beträgt die Auflösungszeit bereits mehr als 2 min 50 sec, meist etwa 3 min. Aber auch bei einem gleichen Verhältnis von Trockenextrakt zu Brausebasis, jedoch mit einem höheren Anteil an alkalischen C02-abspaltenden Bestandteilen, wobei ein pH-Wert von 5,65 resultiert, beträgt die Auflösungszeit bei einer Härte von 7,5 bereits 2 min und darüber.

Es kann erforderlich sein, CO2-abspaltende Alkalibestandteile sowie auch saure Bestandteile in Mischung zu bringen, um einen pH-Wert von 4 bis ca.5,5 zu erreichen; z.B. würde bei Mononatriumcitrat und Natriumhydrogencarbonat ein Verhältnis von 1:0,15 ausreichen, um einen pH-Wert von 4 zu erzielen. Dies würde jedoch nicht ausreichen, um eine entsprechende Auflösungsgeschwindigkeit der Tablette zu erzielen. Gleiches gilt z.B. für Mononatriumtartrat, wo auf 1 Gewichtsteil nur 0,2 Gewichtsteile Natriumbicarbonat erforderlich sind, um einen pH-Wert von 4 zu erreichen, womit aber wiederum nicht die entsprechenden Auflösungseigenschaften erzielt werden können.

Als Extrakte können alle gängigen Pflanzen-Trockenextrakte eingesetzt werden, wie z.B. native, d.h. 100%ige Extrakte, oder aber auch 70%ig-, 60%ig- oder 50%ig sprühgetocknete Extrakte mit z.B. Maltodextrin, Calciumphosphat u.a., bevorzugt aber nicht mit Zucker. Die Mengenangabe der Verhältnisse der Extrakte zur Brausebasis bezieht sich auf den jeweils eingesetzten Extrakt, unabhängig, ob er nativ, d.h. 100%ig ist oder eine andere Konzentration aufweist. Es können auch mehrere Extrakte in Mischung in die erfindungsgemässe Brausezubereitung eingebracht werden, ebenso wie auch die Extrakte mit anderen Wirkstoffen kombiniert werden können. Als Beispiel kann hier eine Echinacea-Brausetablette in einer Dosierung von 250 bis 600 mg Trockenextrakt pro Tablette in Kombination mit Zink angeführt werden.

Die Anwendung der Echinacea erfolgt zur unterstützenden Therapie bei grippalen Infekten und beruht auf den ihr zugesagten immunmodulierenden Eigenschaften. Es zeigte sich bei klinischen Studien eine schnellere Besserung der Symptome eines grippalen Infektes des oberen Respirationstraktes, und die Krankheitsdauer verringerte sich. Grundsätzlich hat zwar auch schon Zink bei diesem Indikationsgebiet Anwendung gefunden, doch konnte im vorliegenden Fall eine unerwartete, synergistische Wirkung festgestellt werden. Zink kann in Form der anorganischen oder organischen Salze eingesetzt werden, wie z.B. als Zinksulfat, -orotat, -glycerophosphat oder -acetat in einer Menge von 5 - 50mg Zinkion pro Dosis.

In die erfindungsgemässe Brausezubereitung können zur Geschmacksverbesserung künstliche Süßstoffe, wie Natriumcyclamat, Saccharin-Natrium, Aspartam, Acesulfam und Neohesperidin, eingebracht werden, vorzugsweise aber - wie oben erwähnt - nicht Zucker, Fructose od.dgl. Hingegen ist der Zusatz von pharmazeutisch oder lebensmittelrechtlich zugelassenen Hilfsstoffen, die die oben erwähnten Bedingungen erfüllen, durchaus erwünscht, wie z.B. von Zuckeralkohol (Mannit ist gegenüber Sorbit bevorzugt!), weniger schnell bzw. leicht löslichen Kohlehydraten, wie z.B. Lactose, oder aber auch von Maltodextrin, etc.

Nach Bedarf können auch - um die Auflösung der Pflanzen-Trockenextrakte zu verbessern - Tenside zugefügt werden. Um die Schaumbildung beim Auflösen zu verhindern und die Auflösung der Tablette zu beschleunigen, können vorteilhafter Weise Anti-Schaummittel in die Formulierung eingebaut werden. Die Tenside oder Anti-Schaummittel können auf den Pflanzen-Trockenextrakt direkt aufgebracht oder in Form einer getrennten Phase der Mischung - z.B. vor dem Verpressen zu Tabletten - zugesetzt werden. Zu diesem Zweck werden Tenside und/oder Anti-Schaummittel auf einen der erfindungsgemäss vorgesehenen, neutralen Hilfs- bzw. Füllstoffe aufgezogen und der Tabletten-Endmischung zugefügt. Zur Herstellung der Anti-Schaummittel-Phase wird das Anti-Schaummittel mittels eines Lösungsmittels oder einer wässrigen Suspension auf einen Füllstoff aufgezogen. Anschliessend wird das Lösungsmittel verdampft, und man fügt diese Phase der Tabletten-Endmischung zu. Die eingebrachte Menge des Anti-Schaummittels hängt weitgehend vom Schaumverhalten des eingesetzten Pflanzenextraktes ab.

Die erfindungsgemäß hergestellte Pflanzenextrakt-Brausezubereitung zeichnet sich durch eine schnelle Auflösung in Wasser (Auflösungszeit bei 17°C unter 3 min) und durch ein geringes Schaumverhalten aus. Die Erfindung wird im folgenden an Hand einiger Beispiele näher - jedoch nicht nur darauf beschränkt - erläutert:

### Beispiel 1 (Silymarin-Trockenextrakt)

Verhältnis Wirkstoff zu Brausebestandteilen 1:4.8, Säure zu Alkali 1:0.8.
Herstellung: 1147 Gewichtsteile Zitronensäure werden auf 60°C erwärmt und mit 24 Gewichtsteilen einer 50%igen wässrigen Trinatriumcitrat-Lösung befeuchtet. Es erfolgt die Zugabe von 917 Gewichtsteilen Natriumhydrogencarbonat, die man unter Rühren anreagieren läßt. Anschließend wird die Mischung mit 16 Gewichtsteilen 50%iger Natriumcitrat-Lösung nochmals granuliert; sodann werden 70 Gewichtsteile Natriumcarbonat zugegeben. Das Granulat wird mittels Vakuum bei einer Temperatur von 50°C getrocknet und auf 2 mm gesiebt. Zu diesem gesiebten Brausegranulat werden 4 Gewichtsteile Aspartam, 20 Gewichtsteile Acesulfam sowie 460 Gewichtsteile Silymarin-Trockenextrakt, weiters 50,1 Gewichtsteile einer Mannit-Simethicon-Phase (0,1 Gewichtsteile Simethicon auf 50 Gewichtsteile Mannit), 40 Gewichtsteile Aroma sowie eine Mischung aus 0,8 Gewichtsteilen Natriumdioctylsulfosuccinat in 15 Gewichtsteilen Mannitol zugefügt.
Das Granulat wird zu Tabletten von 2,85 g verpreßt, die sich in Wasser innerhalb von 80 bis 90 sec auflösen. Die wässrige Lösung zeigt einen pH-Wert von 5,0-5,1.

### Beispiel 2 (Cimicifuga-Trockenextrakt)

Verhältnis Wirkstoff zu Brausebestandteilen: 1:20, Säure zu Alkali: 1:0.69.
Herstellung: 482 Gewichtsteile Zitronensäure werden in einem Vakuumgranulator, dessen Manteltemperatur 90°C beträgt, unter Rühren auf 60°C erwärmt und mit einer Lösung aus 6 Gewichtsteilen Natriumcitrat in 6 Gewichtsteilen Wasser befeuchtet. Sodann werden 304 Gewichtsteile Natriumhydrogencarbonat zugegeben und kontrolliert anreagieren lassen; anschließend werden 28 Gewichtsteile Natriumcarbonat zugemischt. Das resultierende Granulat wird mittels Vakuum bei 50°C getrocknet und auf 2 mm gesiebt. Zu 820 Gewichtsteilen des Brausegranulates werden 41 Gewichtsteile Cimicifuga-Trockenextrakt sowie 100 Gewichtsteile Mannit, 40 Gewichtsteile Aroma und 3 Gewichtsteile Saccharin-Natrium, sowie 30 Gewichtsteile Natriumcyclamat zugefügt.

Das homogen vermischte Granulat wird zu Tabletten mit einem Gewicht von 1,03g verpreßt, die sich in Wasser innerhalb von 50 sec auflösen. Die wässrige Lösung zeigt einen pH-Wert von 4.8.

### Beispiel 3 (Echinacea + Zink)

Verhältnis Wirkstoff zu Brauseanteil 1:5.5, Säure zu Alkali 1:0,69.
Herstellung: 1596 Gewichtsteile Zitronensäure und 41,5 Gewichtsteile Zinksulfat werden in einem beheizbaren Vakuumgranulator unter Rühren auf 60°C erwärmt. Diese Mischung wird mit 8 Gewichtsteilen einer Lösung befeuchtet, die aus 4 Gewichtsteilen Natriumcitrat.2H2O und 4 Gewichtsteilen Wasser besteht. Sodann werden 1005 Gewichtsteile Natriumhydrogencarbonat, 5 Gewichtsteile Aspartam und 9 Gewichtsteile Acesulfam zugegeben und kontrolliert anreagieren gelassen, und schliesslich 92 Gewichtsteile Natriumcarbonat zugegeben.

Das Granulat wird abschließend mittels Vakuum bei 50°C getrocknet und auf 2 mm gesiebt. Dem Granulat werden 500 Gewichtsteile Echinacea-Trockenextrakt 70%ig sowie 110 Gewichtsteile Mannit, weiters 100 Gewichtsteile einer 1%igen Mannit-Simethicon-Phase (auf 99 Gewichtsteile Mannit 1 Gewichtsteil Polysiloxan) sowie 60 Gewichtsteile Aroma zugesetzt, und alles wird homogen vermischt. Die fertige Mischung wird zu Tabletten von je 3,62 g verpreßt, die sich in Wasser innerhalb von 80 bis 90 sec auflösen. Die wässrige Lösung zeigt einen pH-Wert von 4,55 bis 4,70.

### Beispiel 4 (Echinacea-Brausetablette)

Verhältnis Wirkstoff zu Brauseanteil: 1:10, Säure zu Alkali: 1:0,69.
Herstellung: In einen beheizbaren Vakuumgranulator werden 1400 Gewichtsteile Zitronensäure eingebracht, auf 50°C erwärmt und mit 5,5 Gewichtsteilen einer 50%igen Natriumcitrat-Lösung in Wasser befeuchtet. Anschließend werden 500 Gewichtsteile Natriumhydrogencarbonat und 380 Gewichtsteile Kaliumhydrogencarbonat sowie 1 Gewichtsteil Aspartam und 9 Gewichtsteile Acesulfam zugefügt, kontrolliert anreagieren lassen und anschließend 80 Gewichtsteile Natriumcarbonat zugegeben. Das feuchte Granulat wird bei 50°C getrocknet und anschließend auf 2 mm gesiebt. 2360 Gewichtsteile dieser Brausebasis werden mit 235 Gewichtsteilen eines 82%igen, getrockneten Echinacea-Preßsaftes versetzt; weiters werden 70 Gewichtsteile Lactose, 83 Gewichtsteile Mannit sowie 60 Gewichtsteile Aroma zugefügt.

Das Granulat wird zu Tabletten mit einem Tablettengewicht von 2,82 g verpreßt; die sich bei einer Härte von 7 bis 8 in Wasser innerhalb von 80 bis maximal 90 sec auflösen. Die wässrige Lösung zeigt einen pH-Wert von 4,65 bis 4,75.

### Beispiel 5 (Agnus Casti-Brausetablette)

Verhältnis Wirkstoff zu Brauseanteil: 1:22, Säure zu Alkali: 1: 0.5.

Die Herstellung erfolgt analog dem vorgenannten Beispiel, wobei 586 Gewichtsteile Zitronensäure, 278 Gewichtsteile Natriumhydrogencarbonat sowie 16 Gewichtsteile Natriumcarbonat für die Brausebasis zum Einsatz kommen. 880 Gewichtsteile dieser Brausebasis werden mit 40 Gewichtsteilen des Agnus Casti-Trockenextraktes sowie 100 Gewichtsteilen Mannit, 40 Gewichtsteilen Aroma und 3 Gewichtsteilen Saccharin sowie 30 Gewichtsteilen Natriumcyclamat vermischt.

Das Granulat wird zu Tabletten mit einem Tablettengewicht von je 1,05 g verpreßt, die sich in Wasser innerhalb von 60 bis 70 sec auflösen. Die wässrige Lösung zeigt einen pH-Wert von 4,0 - 4,1.

### Beispiel 6 (Echinacea-Brausetablette mit Weinsäure)

Verhältnis Wirkstoff zu Brauseanteil: 1: 15, Säure zu Alkali: 1:0.88.
Herstellung: 1995 Gewichtsteile Weinsäure werden in einem beheizbaren Vakuumgranulator auf 55°C erwärmt und mit 10 Gewichtsteilen Wasser befeuchtet. Sodann werden 1482 Gewichtsteile Natriumhydrogencarbonat zugefügt, kontrolliert anreagieren gelassen und anschließend 273 Gewichtsteile Natriumcarbonat zugegeben. Das Granulat wird mittels Vakuum bei 50°C getrocknet und auf 2 mm gesiebt. Zu dem gesiebten Granulat werden 250 Gewichtsteile Echinacea-Trockenextrakt 70%ig sowie 100 Gewichtsteile Lactose, 9 Gewichtsteile Acesulfam, 5 Gewichtsteile Aspartam, 200 Gewichtsteile einer Mannit-Simethicon-Phase (2 Gewichtsteile Simethicon auf 98 Gewichtsteile Mannit), sowie schliesslich 60 Gewichtsteile Aroma zugefügt und homogen vermischt.

Das Granulat wird zu Tabletten mit 4,37 g verpreßt, die sich in Wasser innerhalb von 110 sec auflösen. Die wässrige Lösung zeigt einen pH-Wert von 4,8.

### Beispiel 7 (Echinacea Brausetablette mit Zitronensäure /Mononatriumcitrat)

Verhältnis Wirkstoff zu Brauseanteil: 1:15, Säure zu Alkali: 1:0.42.

Die Herstellung erfolgt analog dem vorangegangenen Beispiel, wobei für die Brausebasis als Säurekomponenten 1949 Gewichtsteile Zitronensäure und 687 Gewichtsteile Mononatriumcitrat, als Alkalikomponenten 928 Gewichtsteile Natriumhydrogencarbonat und 186 Gewichtsteile Natriumcarbonat eingesetzt werden.

Zu dem gesiebten Brausegranulat von 3750 Gewichtsteilen werden 250 Gewichtsteile Echinacea-Trockenextrakt 70%ig sowie 9 Gewichtsteile Acesulfam, 5 Gewichtsteile Aspartam, 100 Gewichtsteile Mannit-Simethicon-Phase (1 Gewichtsteil Simethicon auf 99 Gewichtsteile Mannit) sowie 60 Gewichtsteile Aroma zugefügt und homogen vermischt.

Das Granulat wird zu Tabletten von 4,17 g verpreßt, die sich in Wasser innerhalb von 70 sec auflösen. Die wässrige Lösung zeigt einen pH-Wert von 4.2.

## Patentansprüche

1. Brausezubereitung in Form von Granulat oder einer Tablette, enthaltend wenigstens einen wasserlöslichen oder zumindest suspendierbaren Pflanzenextrakt und eine Brausebasis aus wenigstens einer festen, essbaren, organischen Säure und/oder deren saurem Salz, sowie wenigstens einem Alkali- und/oder Erdalkalicarbonat bzw. -hydrogencarbonat, dadurch gekennzeichnet, dass die Pflanzenextraktteilchen in inniger Mischung mit einem wasserlöslichen, pharmazeutisch zulässigen Füllstoff vorliegen, der weniger schnell bzw. weniger leicht löslich ist als Zucker, und dass die Brausebasis in ihrer Zusammensetzung und Menge so eingestellt ist, dass sich mit dem Pflanzenextrakt in wässriger Lösung ein pH-Wert von 3.8 bis 5.6, insbesondere von 4.1 bis 5.3, ergibt.

2. Brausezubereitung nach Anspruch 1, dadurch gekennzeichnet, dass der Füllstoff Maltodextrin, Mannit und/oder Lactose ist.

3. Brausezubereitung nach Anspruch 1 oder 2, dadurch gekennzeichnet, dass das Gewichtsverhältnis der sauren zu den CO2-abspaltenden Komponenten der Brausebasis zwischen 1:0.3 und 1:0.9 liegt.

4. Brausezubereitung nach einem der vorhergehenden Ansprüche, dadurch gekennzeichnet, dass das Gewichtsverhältnis des Pflanzen-Trockenextraktes zur Brausebasis zwischen 1:3 und 1:25, bevorzugt zwischen 1:5 und 1:20 liegt.

5. Brausezubereitung nach einem der vorhergehenden Ansprüche, dadurch gekennzeichnet, dass der Pflanzenextrakt Silymarin, Cimicifuga, Echinacea, Efeu oder Agnus Casti ist.

6. Brausezubereitung nach einem der vorhergehenden Ansprüche, dadurch gekennzeichnet, dass sie ausserdem Spurenelemente, insbesondere im Falle des Pflanzenextraktes Echinacea eine wasserlösliche Zinkverbindung, bevorzugt etwa 5 bis etwa 50 mg Zinkion pro Tablette, enthält.

7. Brausezubereitung nach einem der vorhergehenden Ansprüche, dadurch gekennzeichnet, dass sie ausserdem - bevorzugt 0,05 -5, insbesondere 0,1 - 2.0 Gewichtsteile (auf 100 Gewichtsteile des Pflanzenextraktes bezogen) - eines Anti-Schaummittels enthält, insbesondere Dimethicon oder Simethicon.
